**Europäisches Patentamt**

**European Patent Office**

**Office européen des brevets**

(19)

(11) Veröffentlichungsnummer: **0 013 930**
**B1**

(12)

# EUROPÄISCHE PATENTSCHRIFT

(45) Veröffentlichungstag der Patentschrift: **13.06.84**

(21) Anmeldenummer: **80100179.3**

(22) Anmeldetag: **15.01.80**

(51) Int. Cl.³: **G 01 N 33/74,**
**A 61 K 39/385**

(54) Immunogen, Antikörper für ein Thymus-Hormon, markiertes Thymus-Hormon und Verfahren zur Bestimmung dieses Thymushormons.

(30) Priorität: **22.01.79 US 4971**

(43) Veröffentlichungstag der Anmeldung:
**06.08.80 Patentblatt 80/16**

(45) Bekanntmachung des Hinweises auf die Patenterteilung:
**13.06.84 Patentblatt 84/24**

(84) Benannte Vertragsstaaten:
**CH DE FR GB IT NL**

(56) Entgegenhaltungen:
**FR - A - 2 184 847**
**US - A - 4 055 633**
**US - A - 4 079 127**

**Die Akte enthält technische Angaben, die nach dem Eingang der Anmeldung eingereicht wurden und die nicht in dieser Patentschrift enthalten sind.**

(73) Patentinhaber: **THE GEORGE WASHINGTON UNIVERSITY**
**2121 I Street N.W.**
**Washington, D.C. (US)**

(72) Erfinder: **Goldstein, Allan L.**
**2795 28th Street**
**N.W. Washington D.C. 20008 (US)**
Erfinder: **Mc Clure, John E.**
**10151 Mosby Woods Drive**
**Fairfay, Virginia 22030 (US)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80 (DE)**

Courier Press, Leamington Spa, England.

**Beschreibung**

Die Erfindung bezieht sich auf das Gebiet der Immunologie.

Thymosin $\alpha_1$ ist ein hitzestabiles, saures Polypeptid, welches aus 28 Aminosäuren besteht. Dieses Thymushormon wurde aus der Thymosinfraktion 5 von Kälberthymus isoliert und dessen Aminosäurensequenz bestimmt. Thymosin $\alpha_1$ ist eines von mehreren Polypeptiden in der Thymosinfraktion 5 und welches an der Regulierung, Differenzierung und Funktion der thymusabhängigen Lymphocyten (T-Zellen) teilnimmt. Die Isolierung, Charakterisierung und die Verwendung von Thymosin $\alpha_1$ wird in der US-Patentschrift 4,079,127 näher beschrieben.

Die US—Patentschrift 4,055,633 beschreibt einen immunologischen Test für ein Polypeptidhormon des Thymus, welches als Thymopoietin oder Thymin bekannt ist. Im speziellen beschreibt diese Patentschrift einen Radioimmunassay für Thymopoietin, bei dem ein Antikörper verwendet wird, welcher durch ein Immunogen bestehend aus gereinigtem Thymopoietin, welches kovalent an einem immunogenen Träger, wie Rindergammaglobulin gebunden ist, hervorgerufen wurde. Als Kupplungsmittel wurde Glutaraldehyd verwendet. Das in diesem Assay verwendete Antigen ist vorzugsweise $^{125}$Jodthymopoietin.

Es muss beachtet werden, dass Thymopoietin total verschieden ist zu Thymosin $\alpha_1$ bezüglich der Struktur, der Aminosäurezusammensetzung und -Sequenz, der biologischen Aktivität, sowie der physikalischen und immunologischen Eigenschaften.

Schulof et al. beschreiben in Fed. Proc. *32*, 962 (1973) einen Radioimmunassay für eine teilweise gereinigte Thymosinfraktion, d.h. für Thymosinfraktion 6, von welcher man heute weiss, dass sie verschiedene Polypeptide enthält. In diesem Zusammenhang wird auch auf Goldstein et al. in Fed. Proc. *33*, 2053 (1974) verwiesen.

Die vorliegende Erfindung betrifft ein Verfahren zur immunologischen Bestimmung von Thymosin $\alpha_1$. Dieses immunpotenzierende Polypeptidhormon ist ein Bestandteil der Thymosinfraktion 5 und wurde auch im Blut von Säugetieren gefunden. Die Bestimmung des Thymosin $\alpha_1$-Spiegels in biologischen Flüssigkeiten, insbesondere im Serum, liefert einen wortvollen diagnostischen Test zur Erfassung von Immunmangelkrankheiten, Krankheiten des Autoimmunsystems, Krankheiten die immunologisch hervorgerufen wurden sowie neoplastischen Erkrankungen. Weiterhin kann die Thymosin $\alpha_1$-Therapie durch anschliessende Bestimmung des Hormonspiegels im Blut überwacht werden. Die Erfindung betrifft ferner ein Immunogen, dessen aktiver Bestandteil Thymosin $\alpha_1$ ist, einen für Thymosin $\alpha_1$ spezifischen Antikörper und $^{125}$J-Thymosin $\alpha_1$.

Das für die vorliegende Bestimmungsmethode verwendete Immunogen zur Herstellung der Antikörper kann in einfacher Weise durch kovalentes Binden von Thymosin $\alpha_1$ an einem herkömmlichen immunologischen Träger erhalten werden. Die Quelle für das gemäss vorliegender Erfindung zu verwendende Thymosin $\alpha_1$ ist nicht sehr kritisch. Geeignetes Thymosin $\alpha_1$ kann aus Fraktion 5 diverser Säuger gewonnen werden. So z.B. kann Thymosin $\alpha_1$ der Fraktion 5 von Menschen, Rindern, Schafen oder Schweinen verwendet werden. Diese ist möglich, da die Aminosäuresequenzen von Thymosin $\alpha_1$, welche von verschiedenen Säugern hergeleitet ist, homolog ist.

Andererseits kann auch Thymosin $\alpha_1$ verwendet werden, welches nach bekannten Methoden der Peptidsynthese hergestellt wurde. So wird z.B. die Synthese von Thymosin $\alpha_1$ in fester und flüssiger Phase im U.S. Patent No. 4,148,788 beschrieben.

Der in dieser Beschreibung verwendete Ausdruck "immunogenes Trägermaterial" umfasst diejenigen Materialien, welche einerseits die Eigenschaft haben, eine immunogene Antwort in einem Wirtstier hervorzurufen und welche andererseits kovalent an Thymosin $\alpha_1$ gebunden werden können, sie es direkt über die Bindung von Peptid oder Esterbindungen zwischen freien Carboxyl-, Amino- oder Hydroxylgruppen des Thymosins $\alpha_1$ und entsprechenden Gruppen des Trägermaterials oder sei es durch eine Bindung über ein herkömmliches bifunktionelles Kupplungsreagens.

Das kovalente Binden von Thymosin $\alpha_1$ an den immunogenen Träger kann nach bekannten Methoden erfolgen. So z.B. kann man ein Carbodiimid, besonders bevorzugt Dicyclohexylcarbodiimid oder 1-Aethyl-3-(3-dimethylaminopropyl)carbodiimid als Kupplungsreagens verwenden. Bei diesem Kopplungsverfahren ist ein leicht saures Reaktionsmilieu im pH-Bereich von ungefähr 3-6,5 vorzugsweise von etwa 4-6,5 zu verwenden.

Ein geeignetes bifunktionelles Kopplungsreagens ist ein $C_{2-7}$ Dialdehyd, z.B. Glutaraldehyd. Die Kupplung nach dieser Weise kann nach den von S. Avrameas in Immunochemistry *6*, 43 (1969) beschriebenen Bedingungen erfolgen.

Das erhaltene Immunogen kann zwar ohne weitere Reinigung verwendet werden. Es kann aber auch, zur Entfernung des freien Thymosin $\alpha_1$ und des Kopplungsreagens dialysiert werden.

Geeignete Trägermaterialien welche zur Herstellung der Immunogene gemäss vorliegender Erfindung verwendet werden können, umfassen Proteine, natürliche oder synthetische Polymerverbindungen, wie Polypeptide, z.B. Polylysin oder Kopolymere von Aminosäuren, Polysaccharide und dergleichen. Besonders bevorzugte Trägermaterialien sind Proteine und Polypeptide, insbesondere Proteine.

Die Art des Proteins, welches zur Herstellung des Immunogens gemäss vorliegender Erfindung verwendet wird ist nicht kritisch. Beispiele solcher Proteine umfassen Serumproteine von Säugern wie z.B. menschliches Gammaglobulin, menschliches Serumalbumin, Rinderserumalbumin, methyliertes Rinderserumalbumin, Kaninchenserumalbumin, Rindergammaglobulin und Pferdegammaglobulin sowie Proteine von nicht Säugern wie Hemocyanin. Andere geeignete Proteine sind dem Fachmann geläufig.

Das Immunogen gemäss vorliegender Erfindung kann zur Bildung von Thymosin $\alpha_1$ spezifischen Antikörpern verwendet werden, indem man Wirtstieren dieses Immunogen vorzugsweise unter Verwendung eines Adjuvants injiziert. Durch wiederholte Injektionen über eine Zeitperiode können erhöhte Titer erreicht werden. Geeignete Wirtstiere für diesen Zweck umfassen Säugetiere wie Kaninchen, Pferde, Ziegen, Meerschweinchen, Ratten, Kühe, Schafe usw. Die resultierenden Antiseren, enthalten Antikörper, die Thymosin $\alpha_1$ selektiv binden. Bedingt durch die Homologie von Thymosin $\alpha_1$ verschiedener Säuger ist es möglich, Antikörper hervorgerufen durch ein Thymosin $\alpha_1$ eines bestimmten Säugers zur Bestimmung von Thymosin $\alpha_1$ eines anderen Säugers zu verwenden.

Geeignetes markiertes Thymosin $\alpha_1$ für den vorliegenden Immunassay umfasst radiosotop markiertes Thymosin $\alpha_1$ speziell Tritium ($^3$H), Kohlenstoff 14 ($^{14}$C) oder 125 Jod ($^{125}$Jod) Thymosin $\alpha_1$.

Das Tritium kann durch die bekannte Isotopen-Austauschmethode ins Thymosin $\alpha_1$ eingeführt werden. Die Herstellung von $^{14}$C-Thymosin ist leicht möglich durch Einführung von einer oder mehreren käuflich erhältlichen $^{14}$C-markierten Aminosäuren auf einer geeigneten Stufe in der Thymosinsynthese.

Bevorzugt wird das $^{125}$Jod-thymosin $\alpha_1$. Die Abwesenheit von Tyrosin oder Histidin schliesst die Verwendung der direkten Markierung unter Verwendung von Natrium-$^{125}$Jodid und Chloramin-T aus. Es ist dagegen möglich durch Verwendung des Bolton-Hunter Reagens ($^{125}$Jod-jodierter p-Hydroxy-phenylpropionsäure-N-hydroxysuccinimidester; vergl. Biochem. J. *133*, 529 (1973) Thymosin $\alpha_1$ mit $^{125}$Jod zu markieren.

Im Rahmen der vorliegenden Erfindung kann das Thymosin $\alpha_1$ mit anderen Indikatoren markiert werden: Chromophoren, Fluorophoren, Enzymen, Erythrocyten, Latexpartikeln oder zur Ekeltronenspinnresonanz befähigte Gruppen.

Gemäss vorliegender Erfindung können verschiedene Bestimmungsmethoden angewendet werden. Nach einer solchen Methode wird eine bestimmte Menge der zu bestimmenden Probe, mit Thymosin $\alpha_1$-spezifischem Antikörper und markiertem Thymosin $\alpha_1$ vermischt und stehengelassen. Der Antikörperantigenkomplex wird nach bekannten Methoden von ungebundenen Reagenzien abgetrennt, z.B. durch Behandlung mit Ammoniumsulfat, durch Zugabe von Polyäthylenglucol, durch einen Ueberschuss von einem zweiten Antikörper oder einem zweiten Antikörper, der gebunden ist an einen unlöslichen Träger, an Dextran beschichtete Antivkohle und dergleichen. Die Konzentration von markiertem Thymosin $\alpha_1$ wird entweder in der gebundenen oder der ungebundenen Phase bestimmt. Der Thymosin $\alpha_1$ Gehalt der Probe kann dann bestimmt werden durch Vergleich des Spiegels an markierter Substanz mit einer in herkömmlicher hergestellten Standardkurve. Eine geeignete Standardkurve kann dadurch hergestellt werden, dass man bekannte Mengen von Thymosin $\alpha_1$ mit fixen Mengen von markiertem Thymosin $\alpha_1$ und dem gegen Thymosin $\alpha_1$ spezifischen Antikörper mischt und den Bindungsgrad für jede dieser bekannten Mengen bestimmt.

Die vorliegende Erfindung wird durch die nachfolgenden Beispiele illustriert:

Beispiel 1
Erzeugung der Antiseren

A. Kuppeln von synthetischem Thymosin $\alpha_1$ an Hämocyanin mit Hilfe von Glutaraldehyd.

Aequivalente Mengen (trocken gewogen) von Thymosin $\alpha_1$ und Hämocyanin werden in 0,25 M Natriumphosphatpuffer vom pH 7,0 (bis zu einer Konzentration von 2 mg/ml) gelöst. Gleiche Volumina der beiden Proteinlösungen werden gemischt und Glutaraldehyd (25% ige wässrige Lösung) in einer Menge von 1% des Volumens der Proteinmischung beigefügt. Die Mischung wird während 3 Stunden bei Raumtemperatur gerührt. Anschliessend wird das Reaktionsgemisch mit steriler Kochsalzlösung bis zu einer Konzentration von 100 $\mu$g/ml Thymosin $\alpha_1$ verdünnt. Die Lösung des gekoppelten Thymosin $\alpha_1$ wird in aliquote Mengen aufgeteilt und bei −20°C gelagert.

B. Immunisierung und "Booster"-Ueberwachung.

Die Kochsalzlösung des Thymosin $\alpha_1$-Konjugats wird mit einer gleichen Menge von kompletten Freund's Adjuvants, zu welchem eine zusätzliche Menge durch Hitze abgetöteter (M. Tuberkulosis) Bazillen zugegeben wurden, emulgiert. Jedes NZW-Kaninchen (junge Tiere beiderlei Geschlechts von 4—5 Pfund) erhielt 100 $\mu$g Thymosin $\alpha_1$ und 8 ml M. Tuberkulosis H37RA Bazillen in 2 ml Emulsion. Die Immunisierung erfolgte durch Injektion der Emulsion in mehrere intradermale Stellen (20—30 Stellen für jedes Tier) in den Rücken jedes Tiers. Jedes Tier erhielt 4 "Booster"-Serien von Injektionen mit 50 $\mu$g Thymosin $\alpha_1$ in wöchentlichen Intervallen, wiederum an verschiedenen intradermalen Stellen. Die erste Blutentnahme wurde eine Woche nach der Serie von "Booster"-Injektionen vorgenommen und zeigte positive jedoch niedere Titer gegen Thymosin $\alpha_1$. Nach 70 Tagen erfolgte die nächste "Booster"-Injektion von 50 $\mu$g für jedes Kaninchen. Eine Blutentnahme 10 Tage später zeigte einen Anti-thymosin $\alpha_1$ Titer, welcher für einen Radioimmunassay genügend ist.

Die Blutentnahme erfolgte bei den Kaninchen durch Venenpunktur der Mittelohrarterie. Vor der Zentrifugation liess man das Blut während 45 Minuten bei Raumtemperatur gerinnen und über Nacht bei 4°C stehen. Das Serum wurde gesammelt und bei −20°C in Gegenwart von 0,05% Natriumazid gelagert.

C. Titrieren der Antiseren.

Antiseren in serienmässiger Verdünnung wurden mit Radioaktiv markierten Thymosin $\alpha_1$ inkubiert und nach der Doppelantikörpermethode gefällt.

Antiseren mit äquivalenten Titern wurden gesammelt und in kleinen Aliquoten bei −20°C gelagert.

Beispiel 2

Herstellung von radioaktiv markiertem Thymosin $\alpha_1$

Es wurde durch verschiedene elektrophoretische und chromatographische Techniken gezeigt, dass synthetisches Thymosin $\alpha_1$ identisch ist mit dem natürlichen Peptid und eine hohe Reinheit aufweist. Nach der vorerwähnten Methode von Bolton und Hunter wurde iodiertes Tyrosin in das Peptid eingeführt. 5 $\mu$g Thymosin $\alpha_1$ gelöst in 0,1 M Natriumborat von pH 8,5 und einer Konzentration von 0,5 mg/ml werden bei 0°C zu 1 mCi getrocknetem Bolton-Hunter Reagent (New England Nuclear Produkt No. NEX-120H) gegeben. Das Reaktionsgemisch wird während 40 Minuten mit dem Magnetrührer gerührt und dann durch Zugabe von 0,5 ml 0,02 M Glycin verdünnt. Die Abtrennung des iodierten Peptids von nicht eingefügtem Bolton-Hunter Reagent erfolgte durch Gelfiltration an einer Sephadex G-10 Kolonne, welche mit einem Puffer bestehend aus Phosphat gepufferter Kochsalzlösung und 0,1% Gelatine vom pH 7,4 äquilibriert war. Die Fraktionen mit dem Protein zeigen keinen Unterschied in der Immunreaktion und werden daher vereinigt. Bolton-Hunter markiertes Thymosin $\alpha_1$ mit einer spezifischen Radioaktivität von ungefähr 3 $\mu$Ci/$\mu$g war durch 30% ige Trichloressigsäure nur zu 20% fällbar, wogegen durch überschüssiges Antiserum eine 94%-ige Fällung erreicht werden konnte. Das durch die Trennung an der Sephadex G-10 Säule erhaltene radioaktiv markierte Material wurde bei −30°C in kleinen aliquoten Positionen gelagert. Die markierte Substanz wurde nur für Bestimmungen aufgetaut und mit Radioimmunassaypuffer in der Weise verdünnt, dass jedes Bestimmungsprobeglas vom Volumen von 50 $\mu$l eine Radioaktivität von 6000—7000 cpm aufwies.

Beispiel 3

Protokoll des Radioimmunverfahrens

Es wurde eine Standardlösung von synthetischem Thymosin $\alpha_1$ bestehend aus dem Peptid in einer Konzentration von 1 $\mu$g/ml in steriler 0,15 M Natriumchloridlösung hergestellt. Die Standardlösung wurde in aliquoten Anteilen von 1-ml verschlossene Plastikprobegläser gegeben und bei −30°C gelagert und nur aufgetaut zur Herstellung der Standardkurve. Die Standardlösung wurde serienmässig verdünnt in 0,4 ml Radioimmunassaypuffer in 12×75 ml Glasprobegläser. Die Standardkurve bestand auf einem zweifachen Satz an Standardlösung serienmässig verdünnt in je 12 Probegläser.

Unbekannte Serumproben (0,2 ml Serum) wurden in Probegläser pipetiert, welche 0,2 ml Radioimmunassaypuffer enthielten. Zu allen Probegläsern wird Standardantiserum (50 $\mu$l) gegeben ausgenommen die Kontrollen, d.h. die nicht spezifisch bindenden Proben (NSB). Nach raschem Mischen der Lösungen werden die Proben bei 37°C während 1 Stunde inkubiert. Hierauf wird 50 $\mu$l radioaktiv markiertes Material mit einer Radioaktivität von 6000—7000 cpm zugegeben. Es wird erneut gemischt und bei 37°C während 1 Stunde inkubiert. Die Proben werden während 48 Stunden bei 4°C gehalten.

Die Fällung des Immunkomplexes wird nach der Doppelantikörpermethode durchgeführt. Normales Kaninchenglobulin und Ziegenantikaninchenglobulin wird hergestellt durch Fraktionieren von normalem Kaninchenserum und Ziegenentikaninchenglobulin mit Ammoniumsulfat um niedermolekulare Serumbestandteile zu entfernen und das Gammaglobulin anzureichern. Die Seren werden mit Ammoniumsulfat behandelt bis zu einer Konzentration von 30% Sättigung bei 4°C. Die ausgefallenen Proteine werden durch Zentrifugation gesammelt und in einem Anteil von steriler 0,15 M Natriumchloridlösung, welche dem ursprünglichen Volumen von Serum identisch ist wiedergelöst. Die Anteile jedes Reagenses zur optimalen Fällung des radioaktiv markierten Immunkomplexes wurden in einem Titrationsversuch vorbestimmt. Jede neue Charge von normalem Kaninchen IgG und Ziegenantikaninchen IgG muss vor deren Gebrauch im Radioimmunassay standartisiert werden.

Nach Zugabe von normalen Kaninchenglobulin und Ziegenantikanninchengammaglobulin werden die Proben zuerst während 1 Stunde bie 37°C und dann während 16 Stunden bei 4°C inkubiert. Die Abtrennung der ausgefallenen Proteine vom radioaktiven Ueberstand erfolgt durch zentrifugation der Proben bei 2500 Umdrehungen pro Minute während 20 Minuten bei 4°C. Die Ueberstände werden durch Absaugen abgetrennt. Die Niederschläge werden durch Resuspension in 1 ml Radioimmunassaypuffer gewaschen und erneut zentrifugiert. Die Proben werden schliesslich in einen automatischen Gammazähler gebracht, um den Anteil des ratioaktiven Materials zu bestimmen, welches an die Proteinkügelchen gebunden ist.

4

Die 0,2 ml Serumproben werden 3-fach bestimmt. Die durchschnittliche Radioaktivität in cpm für die NSB-Kontrollen wird von jedem Probewert abgezogen.

Die gebundene Radioaktivität der Standard/Serumproben wird mit einem Computerprogramm ausgewertet. Für die Standardkurve wird die Radioaktivität bei Anwesenheit einer bestimmten Menge von Thymosin $\alpha_1$ abzüglich der gebundenen Radioaktivität ohne Thymosin $\alpha_1$ berechnet (Bi/Bo). Die Korrelation als logit (Y) wird berechnet wie auch $\log_{10}$ in Abhängigkeit der Thymosin $\alpha_1$ Standardreihe in Femtomols.

$$\text{Logit }(B_1/B_o)=\ln\frac{B_i/B_o}{(1-B_iB_o)}=Y$$

Die Methode der kleinsten Quadrate wird benützt um die optimale lineare Korrelation der X und Y Werte zu berechnen. Die Steigerung der Y Abschnitte und der Korrelationskoeffizient werden für den output programmiert. Der Gehalt an Substanzen in der Serumprobe die mit Thymosin $\alpha_1$ eine Kreuzreaktion eingeben wird berechnet vom Wert des logit (Bi/Bo) für jede einzelne Probe und von der Steigerung und dem Y Abschnitt von der Standardkurve. Die in 3-fach Bestimmungen erhaltenen Werte ausgedrückt in Femtomols von Thymosin $\alpha_1$ werden weiteren Analysen unterzogen: Bestimmung der Mittelwerte, Standard-Abweichung und prozentuale Standardabweichung. Die Konsentrationsangaben für normal und immunodeffiziente Personen werden in Femtomol/0.2 ml Serum angegeben.

Beispiel 4
Experimentelle Resultate

Das Radioimmunverfahren gemäss Beispiel 3 wird an Serumproben von verschiedenen Mäusestämmen und von 1 Meerschweinchenstamm durch geführt. Die Sensivität der Bestimmungsmethode war genügend um kreuzreagierendes Material in diesen Seren unter Verwendung von menschlichem Thymosin $\alpha_1$ zu bestimmen. Die erhaltenen Daten sind in Tabelle I zusammengefasst:

TABELLE I
Serumspiegel von Thymosin $\alpha_1$ in experimentellen Tiermodellen

| Spezies/Stamm | Serumkonzentration von Thymosin $\alpha_1$ (pMols/ml$\pm$ standard |
|---|---|
| Maus-Balb/c (normal:erwachsen) | 10,6$\pm$2,1 |
| Maus-NZB* (erwachsen) | nicht feststellbar |
| Maus-B/W* (erwachsen) | 2,66$\pm$1,5 |
| Meerschweinchen-Hartley (erwachsen) | 8,15$\pm$3,5 |

* Mäuse welche eine Erkrankung des Autoimmunsystems manifestieren.

Es ist klar von Tabelle I, dass die Serumspiegel von Thymosin $\alpha_1$ in gesunden Balb/c-Mäusen völlig verschieden sind von NZB und B/W-Mäusen (Stämme von Mäusen welche autoimmunähnliche Symptome zeigen).

Gefrorene Serumproben von gesunden Männern verschiedener Alters wurden nach dem Verfahren von Beispiel 3 ebenfalls bestimmt. Die Resultate sind in Tabelle II zusammengefasst:

TABELLE II
Thymosinspiegel in gefrorenen Seren von gesunden Männern

| Altergruppe (Jahre) | Anzahl der untersuchten patienten | Konzentration von Thymosin $\alpha_1$ (pMols/ml$\pm$ standard |
|---|---|---|
| 10—19 | 5 | 1,82$\pm$0,20 |
| 20—29 | 4 | 1,90$\pm$0,57 |
| 30—39 | 7 | 1,07$\pm$0,20 |
| 40—49 | 6 | 1,19$\pm$0,26 |
| 50—59 | 6 | 0,66$\pm$0,06 |

Diese preliminären Daten scheinen anzuzeigen, dass die Thymosin $\alpha_1$-Spiegel im Serum mit dem Alter abhenmen. Es muss allerdings beachtet werden, dass die Zeitdauer, während der die Serumproben gefroren wurden, die Werte von Thymosin $\alpha_1$ beeinflussen können. Die Serumproben

**0 013 930**

dieser gesunden Männer wurden bis zu 3 Jahre lang gefroren gehalten. Es scheint, dass die Werte für diese Proben signifikant tiefer sind als die Werte von frischen, ungefrorenen Serumproben von erwachsenen Männern. Es scheint jedoch, dass das Verhältnis zwischen Alter und Serumspiegel durch die Gefrierung nicht beeinflusst wird.

**Patentansprüche**

1. Immunogen, dadurch gekennzeichnet, daß aktiver Bestandteil Thymosin $\alpha_1$ ist, das kovalent an einen immunogenen Träger gebunden ist.

2. Immunogen nach Anspruch 1, dadurch gekennzeichnet, dass das immunogene Trägermaterial eine Protein ist.

3. Immunogen nach Anspruch 2, dadurch gekennzeichnet, dass das Protein Hemocyanin ist.

4. Immunogen nach Anspruch 3, dadurch gekennzeichnet, dass das Thymosin $\alpha_1$ mit einem $C_{2-7}$ Dialdehyd oder anderen herkömmlichen bifunktionellen Kopplungsreagenzien an das Trägermaterial gebunden ist.

5. Immunogen nach Anspruch 4, dadurch gekennzeichnet, dass das Kopplungsreagens Glutaraldehyd ist.

6. Für Thymosin $\alpha_1$ spezifischer Antikörper, welcher dadurch hergestellt worden ist, dass Wirtstiere mit dem Immunogen gemäss Anspruch 1 immunisiert und die Seren dieser Wirtstiere gesammelt worden sind.

7. $^{125}$I-Thymosin $\alpha_1$.

8. Verfahren zur immunologischen Bestimmung von Thymosin $\alpha_1$ in einer Probe, dadurch gekennzeichnet, dass die Probe mit einer bekannten Menge von markiertem Thymosin $\alpha_1$ und einem Antikörper welcher selektiv mit Thymosin $\alpha_1$ reagiert versetzt wird, dass der entstandene Antigenantikörperkomplex von nicht reagiertem markiertem Thymosin $\alpha_1$ getrennt wird, dass das Mass der Bindung von markiertem Thymosin $\alpha_1$ in diesem Komplex gemessen wird und die Menge an Thymosin $\alpha_1$ in der Probe durch Vergleich des Grads der Bindung mit einer Standardkurve bestimmt wird, welche durch Mischen bekannter Mengen von Thymosin $\alpha_1$ mit bestimmen Mengen von markiertem Thymosin $\alpha_1$ und Antikörper und Bestimmung des Grades der Bindung für jede bekannte Menge von Thymosin $\alpha_1$ hergestellt wurde.

9. Verfahren nach Anspruch 8, dadurch gekennzeichnet, dass ein Radioimmunverfahren und radioaktiv markiertes Thymosin $\alpha_1$ verwendet wird.

10. Verfahren nach Anspruch 9, dadurch gekennzeichnet, dass das radioaktiv markierte Thymosin $\alpha_1$ $^{125}$J-Thymosin $\alpha_1$ ist.

11. Verfahren nach Anspruch 8, dadurch gekennzeichnet, dass der Antigenantikörperkomplex von der Lösung mit Hilfe der Doppelantikörpermethode getrennt wird, wobei eine zweiter Antikörper verwendet wird, welcher gegen das Serum des Wirtstiers, in dem der Thymosin $\alpha_1$ spezifische Antikörper hervorgerufen wurde, immunologisch reaktiv ist.

**Revendications**

1. Immunogène, caractérisé en ce que son composant actif est la thymosine $\alpha_1$, qui est liée de façon covalente sur un support immunogène.

2. Immunogène selon la revendication 1, caractérisé en ce que le support immunogène est une protéine.

3. Immunogène selon la revendication 2, caractérisé en ce que la protéine est l'hémocyanine.

4. Immunogène selon la revendication 3, caractérisé en ce que la thymosine $\alpha_1$ est liée sur le support avec un dialdéhyde en $C_2$ à $C_7$ ou d'autres réactifs de couplage bifonctionnels habituels.

5. Immunogène selon la revendication 4, caractérisé en ce que le réactif de couplage est le glutaraldéhyde.

6. Anticorps spécifique de la thymosine $\alpha_1$ caractérisé en ce qu'on immunise des animaux hôtes avec l'immunogène selon la revendication 1 et en ce qu'on rassemble les sera des ces animaux hôtes.

7. La $^{125}$I-thymosine $\alpha_1$.

8. Procédé de détermination immunologique de thymosine $\alpha_1$ un échantillon, caractérisé en ce qu'on mélange l'échantillon avec une quantité connue de thymosine $\alpha_1$ marquée et un anticorps qui réagit sélectivement avec la thymosine $\alpha_1$, en ce que l'on sépare le complexe antigène-anticorps apparu de la thymosine $\alpha_1$ marquée n'ayant pas réagi, en ce qu'on mesure le degré de liaison de la thymosine $\alpha_1$ marquée dans ce complexe et en ce qu'on détermine la quantié de thymosine $\alpha_1$ dansl'échantillon par comparaison du degré de liaison avec une courbe standard, que l'on prépare en mélangeant des quantités connues de thymosine $\alpha_1$ avec des quantités connues de thymosine $\alpha_1$ marquée et d'anticorps et détermination du degré de liaison pour chaque quantité connue de thymosine $\alpha_1$.

9. Procédé selon la revendication 8, caractérisé en ce qu'on utilise un procédé radioimmunologique et de la thymosine $\alpha_1$ radioactivement marquée.

6

10. Procédé selon la revendication 9, caractérisé en ce que la thymosine $\alpha_1$ radioactivement marquée est le $^{125}$I-thymosine $\alpha_1$.

11. Procédé selon la revendication 8, caractérisé en ce qu'on sépare le complexe antigène-anticorps de la solution à l'aide du procédé de double anticorps, en utilisant un second anticorps lequel est immunologiquement réactif contre le sérum de l'animal hôte dans lequel la thymosine $\alpha_1$ a suscité des anticorps spécifiques.

**Claims**

1. A immunogen, characterized in that its active constituent is thymosin $\alpha_1$ which is covalently bound to an immunogenic carrier.

2. An immunogen according to claim 1, characterized in that the immunogenic carrier material is a protein.

3. An immunogen according to claim 2, characterized in that the protein is hemocyanin.

4. An immunogen according to claim 3, characterized in that the thymosin $\alpha_1$ is bound to the carrier material with a $C_{2-7}$ dialdehyde or another conventional bifunctional coupling reagent.

5. An immunogen according to claim 4, characterized in that the coupling reagent is glutaraldehyde.

6. An antibody specific for thymosin $\alpha_1$, which has been prepared by immunizing host animals with the immunogen according to claim 1 and collecting the serum of these host animals.

7. $^{125}$I-Thymosin $\alpha_1$.

8. A method for the immunological determination of thymosin $\alpha_1$ in a sample, characterized by treating the sample with a known amount of labelled thymosin $\alpha_1$ and an antibody which reacts selectively with thymosin $\alpha_1$, separating the resulting antigen-antibody complex from unreacted labelled thymosin $\alpha_1$, measuring the extent of the binding of labelled thymosin $\alpha_1$ in this complex and determining the amount of thymosin $\alpha_1$ in the sample by comparing the degree of binding with a standard curve which has been prepared by mixing known amounts of thymosin $\alpha_1$ with specific amounts of labelled thymosin $\alpha_1$ and antibody and determining the degree of binding for each known amount of thymosin $\alpha_1$.

9. A method according to claim 8, characterized in that a radioimmune method and radioactivity labelled thymosin $\alpha_1$ are used.

10. A method according to claim 9, characterized in that the radioactively labelled thymosin $\alpha_1$ is $^{125}$I-thymosin $\alpha_1$.

11. A method according to claim 8, characterized in that the antigen-antibody complex is separated from the solution with the aid of the double antibody method, there being used a second antibody which is immunologically reactive towards the serum of the host animal in which the antibody specific to the thymosin $\alpha_1$ has been produced.